(19) Europäisches Patentamt
European Patent Office
Office européen des brevets

(11) Veröffentlichungsnummer: **0 076 784**
**B1**

(12) # EUROPÄISCHE PATENTSCHRIFT

(45) Veröffentlichungstag der Patentschrift: **05.08.87**

(51) Int. Cl.⁴: **C 10 M 135/20,** C 07 D 295/22, C 07 C 161/00

(21) Anmeldenummer: **82810408.3**

(22) Anmeldetag: **04.10.82**

(54) **Schmiermittelzusammensetzungen und Schmiermittel-Additive.**

(30) Priorität: **06.10.81 CH 6416/81**

(43) Veröffentlichungstag der Anmeldung:
**13.04.83 Patentblatt 83/15**

(45) Bekanntmachung des Hinweises auf die Patenterteilung:
**05.08.87 Patentblatt 87/32**

(84) Benannte Vertragsstaaten:
**DE FR GB IT**

(56) Entgegenhaltungen:
**US - A - 2 343 524**
**US - A - 2 649 470**
**US - A - 3 394 185**

(73) Patentinhaber: **CIBA-GEIGY AG, Klybeckstrasse 141, CH-4002 Basel (CH)**

(72) Erfinder: **Dubas, Henri, Dr., Kuntmattring 16, CH-4107 Ettingen (CH)**
Erfinder: **Kirchmayr, Rudolf, Dr., Ettingerstrasse 9, CH-4147 Aesch (CH)**

**EP 0 076 784 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung von 2 N-Atome enthaltenden Polysulfiden als Schmiermittel-Additive.

Mineralischen und synthetischen Schmierstoffen werden im allgemeinen verschiedene Zusatzstoffe zur Verbesserung ihrer Gebrauchseigenschaften beigegeben. Insbesondere besteht der Bedarf an Additiven, welche die zu schmierenden Vorrichtungen vor Reibungsabnutzung schützen sollen. An solche Verschleissinhibitoren wird die Anforderung gestellt, dass sie das Lasttragevermögen des Schmierstoffs erhöhen und nicht korrodierend auf die zu schützenden Metallteile wirken. Bekannte Schmiermittelzusätze sind geschwefeltes Spermöl und geschwefelte Olefine, z.B. gemäss DT-OS 2 166 893 und DT-OS 2 606 101. Die Hochdruckeigenschaften von solchen Additive enthaltenden Schmiermitteln sind vielfach nicht zufriedenstellend. Insbesondere ist die thermische Stabilität ungenügend und es tritt Korrosion gegen Kupfer und Eisen ein. Weiter werden in der US-A 2 343 524 Dimorpholinosulfide als Zusätze für Benzin, Schmieröle und Gummi beschrieben.

Die Aufgabe der vorliegenden Erfindung liegt nun darin, Schmiermittelmischsysteme beizubringen, welche die Nachteile der genannten bekannten Systeme nicht aufweisen.

Gegenstand der Erfindung sind Schmiermittelzusammensetzungen enthaltend:

a) einem mineralischen und/oder synthetischen Basisöl

b) einer oder mehrerer Verbindungen der Formel I

$$A-(S)_n-G \quad (I),$$

in der n eine Zahl von 2 bis 6 bedeutet, und A und G gleich oder verschieden sind und

$$\genfrac{}{}{0pt}{}{R^1}{R^2}{>}N-$$

bedeuten, worin $R^1$ und $R^2$ gleich oder verschieden sind und gegebenenfalls durch $-Cl$, $-ZR^5$, $-ZCN$, $-NCZ$, $-CZZ^1R^5$, $-ZCZ^1R^5$, $-CZNR^7R^8$ $-NR^7R^8$ oder $-CZZ^1E$ substituiertes, geradkettiges oder verzweigtes $C_1$–$C_{24}$-Alkyl, $C_3$–$C_{18}$-Alkenyl, $C_3$–$C_{12}$-Cycloalkyl oder Aryl mit 6 bis 10 C-Atomen im Kern oder Aralkyl mit bis zu 30 C-Atomen ohne Berücksichtigung der Substituenten darstellen, wobei Z und $Z^1$ unabhängig voneinander O, S, $S_2$, SO, $SO_2$ oder $SO_3$ und $R^5$ Wasserstoff oder gegebenenfalls durch $-Cl$, $-ZCN$, $-NCZ$ oder $-CN$ substituiertes $C_1$–$C_{18}$-Alkyl oder $C_3$–$C_{18}$-Alkenyl bedeuten und E Na, K oder $-NR^7R^8R^9$ darstellen, wobei $R^7$ bis $R^9$ wie $R^5$ definiert sind und gleich oder verschieden sein können,

oder in der A und/oder G einen heterocyclischen Rest mit 5 bis 12 Ringgliedern darstellen, wobei der Kern gesättigt oder ungesättigt ist und als Bindungsatom zur $-(S)_n$-Gruppe ein N-Atom enthält, und welcher zusätzlich auch noch O, S oder ein zweites N als Heteroatom und bis zu 4 mal $R^1$ bzw. $R^2$ am Kern als Substituenten enthalten kann, mit der Massgabe, dass, wenn A Morpholino oder methyl bzw. äthyl substituiertes Morpholino bedeutet, G nicht ebenfalls Morpholino oder methyl oder äthyl substituiertes Morpholino sein kann, und

c) gegebenenfalls weitere Schmiermittel-Additive,

wobei das Basisöl (a) 0,001 bis 5 Gew.-%, bezogen auf Basisöl, der Verbindung der Formel I enthält.

$R^1$ und $R^2$ können folgende Alkylreste bedeuten: z.B. Methyl, iso-Propyl, n-Butyl, sec.-Butyl, tert.-Amyl, 2-Äthylhexyl, n-Octyl. tert.-Octyl, n-Dodecyl, 1,1,7,7-Tetramethyl-octyl, n-Octadecyl. Bevorzugt ist 2-Äthylhexyl.

$R^1$ und $R^2$ können Cyclohexyl bedeuten, wie z.B. Cyclopropyl, Cyclopentyl, Cyclohexyl, Cycloheptyl, Cyclooctyl oder Cyclododecyl. Bevorzugt sind Cyclopentyl und Cyclohexyl.

Stehen $R^1$ Und $R^2$ für Aralkyl, so kommen Benzyl, Phenyläthyl und 2-Phenyl-iso-propyl in Frage.

Stehen $R^1$ und $R^2$ für Alkaryl, so kann es beispielsweise Tolyl, Xylyl, 2,6-Diäthylphenyl oder 4-tert.-Butylphenyl sein.

Als Aryl stehen für $R^1$ und $R^2$ beispielsweise Phenyl, α-Naphthyl oder β-Naphthyl.

Stehen A und/oder G für eine heterozyklische Gruppe, so kann diese beispielsweise Morpholino, Piperazino, Piperidino, Pyrrolidino, Hexamethylenimino, Imidazolidino, Imidazo oder Pyrrolo sein.

Als Substituenten der obigen Gruppen $R^1$ und $R^2$ oder der heterozyklischen Gruppen A und/oder G kommen beispielsweise in Frage: Methyl, Äthyl, Isopropyl, n-Butyl, Vinyl, Allyl, Chlor, Mercapto, Methylthio, Methylsulfoxido, Dodecylthio, Dodecyldithio, Sulfo, Sulfonato, Sulfamoyl, Methoxysulfinyl, Hydroxy, Methoxy, Äthoxy, Allyloxy, n-Octyloxy, 3,6,9,12,15-Pentaoxa-heptadecyloxy, Cyano, Cyanato, Thiocyanato, Isothiocyanato, Methoxycarbonyl, Isooctyloxycarbonyl, Carboxy, Natriumcarboxylato, Acetoxy, 2-Äthylhexanoyloxy, Butanoyl, Dimethylamino, Bis-(2,2')dihydroxyäthylamino, Didodecylamino, wobei die Erfindung in keiner Weise durch die aufgezählten Substituenten begrenzt ist.

Bevorzugt sind solche Schmiermittelzusammensetzungen, welche Verbindungen (b) der Formel I enthalten, in der A und G gleich oder verschieden sind und

$$\genfrac{}{}{0pt}{}{R^1}{R^2}{>}N-$$

bedeuten, worin $R^1$ und $R^2$ gleich oder verschieden sind und gegebenenfalls durch $-Cl$, $-ZR^5$, $-ZCN$, $-NCZ$, $-CZZ^1R^5$, $-ZCZ^1R^5$, $-CZNR^7R^8$, $-NR^7R^8$ oder $-CZZ^1E$ substituiertes, geradkettiges oder verzweigtes $C_1$–$C_{24}$-Alkyl bedeuten, und wobei für den Fall, dass nur A die obige Bedeutung hat, G eine heterocyclische 5- bis 12-Ring-Gruppierung wie vorher definiert darstellt.

Bevorzugt sind weiter solche Schmiermittelzusammensetzungen, welche Verbindungen (b) der

Formel I enthalten, in der A und G gleich oder verschieden sind und

$$\begin{matrix}R^1\\R^2\end{matrix}>N-$$

bedeuten, worin $R^1$ und $R^2$ gleich oder verschieden sind und ebenso wie auch $R^5$ gegebenenfalls durch –Cl, $-ZR^5$, ZCN, –NCZ, $-CZZ^1R^5$, $NR^7R^8$, $-ZCZ^1R^5$, $-CZNR^7R^8$ oder $-CZZ^1E$ substituiertes, geradkettiges oder verzweigtes $C_1$–$C_{18}$-Alkyl bedeuten, und wobei für den Fall, dass nur A die obige Bedeutung hat, G eine heterocyclische 5- bis 12-Ringgruppierung wie vorher definiert darstellt.

Bevorzugt sind weiter solche Schmiermittelzusammensetzungen, welche Verbindungen (b) der Formel I enthalten, in der A Morpholino bedeutet, und G

$$\begin{matrix}R^1\\R^2\end{matrix}>N-$$

bedeutet, wobei $R^1$ und $R^2$ irgendeine der vorher genannten Definition aufweisen.

Bevorzugt sind ebenfalls Schmiermittelzusammensetzungen, welche solche Verbindungen (b) der Formel I enthalten, in der n die Zahl 2 bedeutet.

Besondere Vorzugsformen der Erfindung stellen die folgenden 3 Schmiermittelzusammensetzungen dar:

1. Schmiermittelzusammensetzungen welche solche Verbindungen (b) der Formel I enthalten, in der A und G gleich oder verschieden sind und

$$\begin{matrix}R^1\\R^2\end{matrix}>N-$$

bedeuten, worin $R^1$ und $R^2$ gleich oder verschieden sind und geradkettiges oder verzweigtes, nicht substituiertes $C_1$–$C_{18}$-Alkyl bedeuten.

2. Schmiermittelzusammensetzungen welche solche Verbindungen (b) der Formel I enthalten, in der A und G

$$\begin{matrix}R^1\\R^2\end{matrix}>N-$$

bedeuten, worin $R^1$ und $R^2$ gleiche Reste darstellen und $C_5$–$C_{15}$-Alkyl bedeuten.

3. Schmiermittelzusammensetzungen welche solche Verbindungen (b) der Formel I enthalten, in der A und G gleich sind und eine gleiche der vorher definierten 5- bis 12-Ringgruppierung bedeuten.

Die erfindungsgemäss verwendeten Verbindungen der Formel I sind bekannt und in dem US-Patent 3 359 247 als Vulkanisierungsmittel und Vulkanisationsbeschleuniger beschrieben. Die Herstellung erfolgt nach bekannten Verfahren, auf die hier nicht ausführlich eingegangen wird. Beispielsweise kann ein sekundäres Amin mit Dischwefeldichlorid und einer Base im Molverhältnis 2:1:2 in einem Lösungsmittel umgesetzt werden.

Beispiele für Verbindungen der Formel I sind folgende Substanzen:

1. Bis-(di-isooctylamino)disulfid
2. Bis-(di-cyclohexylamino)disulfid
3. Bis-(N-phenyl-N-äthylamino)-disulfid
4. $[CH_3CH_2-(OCH_2CH_2)_5]_2$-N-S-S-N$[(CH_2CH_2O)_5CH_2CH_3]_2$
5. $(H_{25}C_{12}SCH_2CH_2)$N-S-S-N-$(CH_2CH_2SC_{12}H_{25})_2$
6. 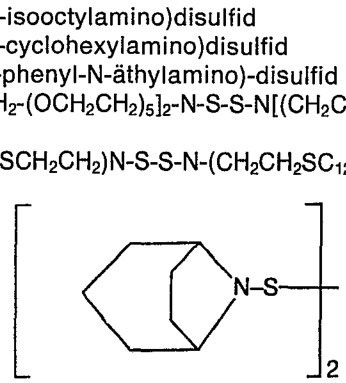
7. Bis-(di-2-acetoxyäthylamino)-disulfid
8. Bis-(2-trichlormethoxycarbonyl-pyrrolidino)-disulfid
9. Bis-(di-p-methylpiperazino)-disulfid
10. Bis-(di-piperidino)-disulfid
11. Bis-(dicyclohexylamino)-tetrasulfid
12. (Dicyclohexylamino-morpholino)-disulfid
13. Bis-(imino diessigsäure)dithio-tetra-n-butylester

Bei den weiteren Schmiermittel-Additiven (c), die zwecks Verbesserung gewisser Gebrauchseigenschaften gegebenenfalls der erfindungsgemässen Schmiermittelzusammensetzung zugegeben werden, handelt es sich bevorzugt um Antioxidantien, Metallpassivatoren, Rostinhibitoren, Viskositätsindex-Verbesserer, Stockpunkterniedriger und Dispersants/Detergents.

Beispiele für Antioxidantien sind:

a) Alkylierte und nicht-alkylierte aromatische Amine und Mischungen davon, z.B.:
Dioctyldiphenylamin, Mono-t-octylphenyl-α- und β-naphthylamine, Phenotriazin, Dioctylphenothiazin, Phenyl-α-naphthylamin, N,N′-Di-sec.-butyl-p-phenylendiamin.

b) Sterisch gehinderte Phenole, z.B.
2,6-Di-tert.-butyl-p-cresol,
4,4′-Bis-(2,6-diisopropylphenol),
2,4,6-Triisopropylphenol,
2,2′-Thio-bis-(4-methyl-6-tert.-butylphenol),
4,4′-Methylen-bis-(2,6-di-tert.-butylphenol).

c) Alkyl-. Aryl- oder Alkaryl-phosphite, z.B.: Trinonylphosphit, Triphenylphosphit, Diphenyldecylphosphit.

d) Ester von Thiodipropionsäure oder Thiodiessigsäure, z.B.: Dilaurylthiodipropionat oder Dioctylthiodiacetat.

e) Salze von Carbamin- und Dithiophosphorsäuren, z.B.: Antimondiamyldithiocarbamat, Zinkdiamyldithiophosphat.

f) Kombination von zwei oder mehr Antioxidantien der obigen, z.B.: ein alkyliertes Amin und ein sterisch gehindertes Phenol.

Beispiele für Metallpassivatoren sind

a) für Kupfer, z.B. Benzotriazol, Tetrahydrobenzotriazol, 2-Mercaptobenzotriazol, 2,5-Dimercaptothiadiazol, Salicyclidenpropylendiamin, Salze von Salicylaminoguanidin.

b) für Blei, z. B.: Sebacinsäurederivate, Chinizarin, Propylgallat,

c) Kombination von zwei oder mehr der obigen Additive.

Beispiele für Rost-Inhibitoren sind:

a) Organische Säuren, ihre Ester, Metallsalze und Anhydride, z. B.: N-Oleoyl-sarcosin, Sorbitanmono-oleat, Blei-naphthenat, Dodecenylbernsteinsäure-anhydrid.

b)Stickstoffhaltige Verbindungen z. B.:

I. Primäre, sekundäre oder tertiäre aliphatische oder cycloaliphatische Amine und Amin-Salze von organischen und anorganischen Säuren, z. B. öllösliche Alkylammoniumcarboxylate.

II. Heterocyclische Verbindungen z. B.:

Substituierte Imidazoline und Oxazoline.

c) Phosphorhaltige Verbindungen z. B.:

Aminsalze von Phosphorsäurepartialestern,

d) Schwefelhaltige Verbindungen z. B.:

Barium-dionylnaphthalin-sulfonate, Calciumpetroleumsulfonate,

e) Kombinationen von zwei oder mehr der obigen Additive.

Beispiele der Viskositätsindex-Verbesserer sind z. B.:

Polymethacrylate, Vinylpyrrolidon/Methacrylat-Copolymere, Polybutene, Olefin-Copolymere, Styrol/Acrylat-Copolymere.

Beispiele für Stockpunkterniedriger sind z. B.:

Alkylierte Naphthaline, alkylierte Phenole, Polymethacrylate.

Beispiele für Detergentien und Dispersants

sind Polyalkenylbernsteinsäureimide, öllösliche Metallseifen wie Ca-, Ba-, Mg- und Al-Carboxylate, -Phenole oder -Sulfonate.

Beispiele für andere Verschleissschutz-Additive sind z. B.:

Schwefel und/oder Phosphor und/oder Halogen enthaltende Verbindungen, wie geschwefelte vegetabilische Öle, Zinkdialkyldithiophosphate, Tritolyl-phosphat, chlorierte Paraffine, Alkyl- und Aryldisulfide.

Die Konzentration der weiteren Schmiermittel-Additiven (c) in den erfindungsgemässen Schmiermittelzusammensetzungen ist sehr unterschiedlich, je nachdem, welcher Typ verwendet wird. Dem Fachmann sind diese Konzentrationen aber sehr geläufig, so dass auf die Angaben derselben hier verzichtet werden kann.

Weiterer Gegenstand der Erfindung ist auch die Verwendung von Verbindungen der Formel I als Schmiermittel-Additive.

Werden mehrere Verbindungen der Formel I gleichzeitig erfindungsgemäss verwendet, so werden diese Verbindungen einzeln oder im Gemisch dem Basisöl zugemischt.

Die Verbindungen der Formel I wirken schon in sehr geringen Mengen als Hochdruck-Zusätze in Schmiermitteln. So zeigen mineralische und synthetische Schmieröle, sowie deren Gemische, welche mit 0,001 bis 5 Gew.-% bezogen auf das Schmiermittel, und vorzugsweise 0,02 bis 3% einer Verbindung der Formel I ausgestattet sind, ausgezeichnete Hochdruck-Schmiereigenschaften, welche durch stark reduzierte Abnutzungserscheinungen der zu schmierenden Reibpartner deutlich werden. Die in Frage kommenden Schmieröle sind dem Fachmann geläufig und z. B. im «Schmiermittel Taschenbuch» (Hüthig Verlag, Heidelberg, 1974) beschrieben.

Die folgenden Beispiele erläutern die Erfindung.

Beispiele

Die Herstellung der Produkte erfolgt in analoger Weise, wie sie nachfolgend für das Additiv Nr. 1 beschrieben wird. (Dabei sind Teile Gewichtsteile). Die Nummern der Verbindungen entsprechen den Angaben auf S. 6 und 7.

Herstellung des Additivs (b) der Formel I Nr. 1

10 Teile Diisooctylamin werden in 67 Teile Äther vorgelegt und innerhalb 50 Minuten bei 1–3 °C zuerst mit der Hälfte einer Lösung von 28 Teilen Dischwefeldichlorid in 10 Teilen Äther, dann mit 3 Volumenteilen einer 25%igen wässrigen Natriumhydroxidlösung versetzt. Anschliessend werden bei 1–3 °C innert 40 Minuten der Rest der Dischwefeldichloridlösung sowie nochmals 3 Volumenteile 25%ige Natriumhydroxidlösung gleichzeitig zugegeben. Nach Abtrennung der wässrigen Phase wird die organische Phase mit 25 Teilen Wasser in drei Portionen gewaschen, dann getrocknet und eingedampft. Man erhält 11 Teile eines gelben Öles.

Beispiele 1–7

Es werden 6 erfindungsgemässe Schmiermittelzusammensetzungen nach der «Tentative Method IP 239/69» (extreme pressure and wear lubricant test for oils and greases, four ball-machine) unter Verwendung des Shell-Vierkugel-Apparates untersucht.

Die Versuchsergebnisse sind in der Tabelle 1 zusammengestellt.

Beispiel 8:

Das kommerzielle Gemisch primärer Amine, das im Handel unter dem Namen PRIMENE® 81-R (Rohm + Haas) erhältlich ist, wird mit Acrylnitril bei erhöhter Temperatur zu einem Gemisch sekundärer Amine umgesetzt. Dieses Produkt wird dann mit Dischwefeldichlorid umgesetzt; die Bedingungen sind denen gleich, die für die Herstellung des Additivs (b) der Formel I Nr. 1 angegeben sind, ausser dass als Lösungsmittel ein Siedegrenzebenzin verwendet wird.

Das Produkt ergibt auf dem Shell-Vierkugel-Apparat folgende Resultate:

| | |
|---|---|
| Konzentration des Additivs: | 1% |
| Basisöl: | Vitrea 41® |
| ISL: | 80 kg |
| WL: | 220 kg |
| WSD: | 0,5 mm |

Tabelle 1

| Beisp. | Basisöl (a) | Additiv (b) der Formel I Nr. | Konz. (Gew.-%) | ISL (kg) | WL (kg) | WSD (mm) |
|---|---|---|---|---|---|---|
| 1 | Vitrea 41® | ohne | – | 60 | 145 | 1,2 |
| 2 | Vitrea 41® | 1 | 1% | 70 | 240 | 0,5 |
| 3 | Vitrea 41® | 2 | 1% | – | 240 | 0,5 |
| 4 | Vitrea 41® | 3 | 1% | – | 240 | 0,5 |
| 5 | Vitrea 41® | 9 | 1% | – | 260 | 0,5 |
| 6 | Vitrea 41® | 10 | 1% | – | 300 | 0,6 |
| 7 | Vitrea 41® | 13 | 1% | – | 220 | 0,5 |

I.S.L. = Initial Seizure Load: Das ist die Last, bei der der Oelfilm innerhalb einer Belastungsdauer von 10 Sekunden zusammenbricht.

W.L. = Weld Load (Schweisslast). Das ist die Last, bei der die 4 Kugeln innerhalb von 10 Sekunden zusammenschweissen.

W.S.D. = Wear Scar Diameter. Das ist der mittlere Verschleissdurchmesser bei einer Belastung von 40 kg während 1 Stunde.

Vitrea 41® ist ein Basisöl der Firma Shell.

Beispiel 9:
Es wird die Korrosivität von Vitrea 41®, das 1 Gew.-% des Additivs Nr. 1 enthält nach DIN 51585 A bestimmt. Ergebnis: Korrosion O–A; Bedeutung: keine Korrosion.

**Patentansprüche**

1. Schmiermittelzusammensetzungen enthaltend:
a) ein mineralisches und/oder synthetisches Basisöl,
b) eine oder mehrere Verbindungen der Formel I

$$A-(S)_n-G \qquad (I),$$

in der n eine Zahl von 2 bis 6 bedeutet, und A und G gleich oder verschieden sind und

$$^{R^1}_{R^2}{>}N-$$

bedeuten, worin $R^1$ und $R^2$ gleich oder verschieden sind und gegebenenfalls durch –Cl, $-ZR^5$, –ZCN, –NCZ, $-CZZ^1R^5$, $-ZCZ^1R^5$, $-CZNR^7R^8$ $-NR^7R^8$ oder $-CZZ^1E$ substituiertes, geradkettiges oder verzweigtes $C_1$–$C_{24}$-Alkyl, $C_3$–$C_{18}$-Alkenyl, $C_3$–$C_{12}$-Cycloalkyl oder Aryl mit 6 bis 10 C-Atomen im Kern oder Aralkyl mit bis zu 30 C-Atomen ohne Berücksichtigung der Substituenten darstellen, wobei Z und $Z^1$ unabhängig voneinander O, S, $S_2$, SO, $SO_2$ oder $SO_3$ und $R^5$ Wasserstoff oder gegebenenfalls durch –Cl, –ZCN, –NCZ oder –CN substituiertes $C_1$–$C_{18}$-Alkyl oder $C_3$–$C_{18}$-Alkenyl bedeuten und E Na, K oder $-NR^7R^8R^9$ darstellen, wobei $R^7$ bis $R^9$ wie $R^5$ definiert sind und gleich oder verschieden sein können,
oder in der A und/oder G einen heterocyclischen Rest mit 5 bis 12 Ringgliedern darstellen, wobei der Kern gesättigt oder ungesättigt ist und als Bindungsatom zur $-(S)_n$-Gruppe ein N-Atom enthält, und welcher zusätzlich auch noch O, S oder ein zweites N als Heteroatom und bis zu 4 mal $R^1$ bzw. $R^2$ am Kern als Substituenten enthalten kann, mit der Massgabe, dass, wenn A Morpholino oder methyl bzw. äthyl substituiertes Morpholino bedeutet, G nicht ebenfalls Morpholino oder methyl bzw. äthyl substituiertes Morpholino sein kann, und
c) gegebenenfalls weitere Additive, wobei das Basisöl (a) 0,001 bis 5 Gew.-%, bezogen auf Basisöl, der Verbindung der Formel I enthält.

2. Schmiermittelzusammensetzungen nach Anspruch 1, dadurch gekennzeichnet, dass in Formel I A und G gleich oder verschieden sind und

$$^{R^1}_{R^2}{>}N-$$

bedeuten, worin $R^1$ und $R^2$ gleich oder verschieden sind und gegebenenfalls durch –Cl, $-ZR^5$, –ZCN, –NCZ, $-CZZ^1R^5$, $-ZCZ^1R^5$, $-CZNR^7R^8$, $-NR^7R^8$ oder $CZZ^1E$ substituiertes, geradkettiges oder verzweigtes $C_1$–$C_{24}$-Alkyl bedeuten
und wobei für den Fall, dass nur A die obige Bedeutung hat, G eine heterocyclische 5- bis 12-Ring-Gruppierung wie in Anspruch 1 definiert darstellt.

3. Schmiermittelzusammensetzungen nach Anspruch 1, dadurch gekennzeichnet, dass in Formel I A Morpholino bedeutet, und G

$$^{R^1}_{R^2}{>}N-$$

bedeutet, wobei $R^1$ und $R^2$ wie in Anspruch 1 definiert sind.

4. Schmiermittelzusammensetzungen nach Anspruch 1, dadurch gekennzeichnet, dass in Formel 1 A und G gleich oder verschieden sind und

$$^{R^1}_{R^2}{>}N-$$

bedeuten, worin $R^1$ und $R^2$ gleich oder verschieden sind und ebenso wie auch $R^5$ gegebenenfalls durch –Cl, –$ZR^5$, ZCN, –NCZ, –$CZZ^1R^5$, –$NR^7R^8$, –$ZCZ^1R^5$, –$CZNR^7R^8$ oder –$CZZ^1E$ substituiertes, geradkettiges oder verzweigtes $C_1$–$C_{18}$-Alkyl bedeuten,
und wobei für den Fall, dass nur A die obige Bedeutung hat, G eine heterocyclische 5- bis 12-Ring-Gruppierung wie in Anspruch 1 definiert darstellt.

5. Schmiermittelzusammensetzungen nach Anspruch 1, dadurch gekennzeichnet, dass in Formel I A und G gleich oder verschieden sind und

$$\begin{matrix}R^1\\R^2\end{matrix}{>}N-$$

bedeuten, worin $R^1$ und $R^2$ gleich oder verschieden sind und geradkettiges oder verzweigtes, nicht substituiertes $C_1$–$C_{18}$-Alkyl bedeuten.

6. Schmiermittelzusammensetzungen nach Anspruch 5, dadurch gekennzeichnet, dass in Formel I A und G

$$\begin{matrix}R^1\\R^2\end{matrix}{>}N-$$

bedeuten, worin $R^1$ und $R^2$ gleiche Reste darstellen und $C_5$–$C_{15}$-Alkyl bedeuten.

7. Schmiermittelzusammensetzungen nach Anspruch 1, dadurch gekennzeichnet, dass in Formel I A und G gleich sind und eine gleiche der gemäss Anspruch 1 definierten 5- bis 12-Ring-Gruppierungen bedeuten.

8. Schmiermittelzusammensetzungen nach Anspruch 1, dadurch gekennzeichnet, dass in Formel I n die Zahl 2 bedeutet.

9. Schmiermittelzusammensetzungen nach Anspruch 1, dadurch gekennzeichnet, dass sie als weiteres Schmiermittel-Additiv (c) ein solches aus der Reihe Antioxidantien, Metalldesaktivatoren, Rostschutzmittel, Dispersants, Detergentien, Viskositätsindexverbesserer oder Stockpunkterniedriger oder ein Gemisch mehrerer dieser Substanzen enthalten.

10. Verwendung von Verbindungen der Formel I gemäss Anspruch 1 als Schmiermittel-Additive.

11. Verwendung mehrerer Verbindungen der Formel I gemäss Anspruch 10 als Schmiermittel-Additive, wobei die Verbindungen einzeln oder im Gemisch dem Basisöl zugemischt werden.

## Claims

1. A lubricant composition containing
a) a mineral and/or synthetic base oil;
b) one or more compounds of the formula I

$$A\text{–}(S)_n\text{–}G \qquad (I)$$

wherein n is an integer from 2 to 6 and A and G are the same or different and represent

$$\begin{matrix}R^1\\R^2\end{matrix}{>}N-$$

wherein $R^1$ and $R^2$ are the same or different and are straight chain or branched $C_1$–$C_{24}$alkyl, $C_3$–$C_{18}$alkenyl, $C_3$–$C_{12}$cycloalkyl or aryl containing 6 to 10 carbon atoms in the nucleus, or aralkyl containing up to 30 carbon atoms, with the indicated number of carbon atoms not including those carbon atoms in the following substituents, each of which radicals is unsubstituted or substituted by –Cl, –$ZR^5$, –ZCN, –NCZ, –$CZZ^1R^5$, –$ZCZ^1R^5$, –$CZNR^7R^8$, –$NR^7R^8$, or –$CZZ^1E$, each of Z and $Z^1$ independently of the other is –O–, –S–, –$S_2$, –SO–, –$SO_2$, or –$SO_3$, $R_5$ is hydrogen or $C_1$–$C_{18}$alkyl or $C_3$–$C_{18}$alkenyl, each unsubstituted or substituted by –Cl, –ZCN, –NCZ or –CN, and E is Na, K or –$NR^7R^8R^9$, in which $R^7$ to $R^9$ are as defined for $R^5$ and may be the same or different; or wherein A and/or G are a heterocyclic radical containing 5 to 12 ring members and the nucleus of which is saturated or unsaturated and contains a N atom as the atom linking it to the –$(S)_n$– group, which radical may additionally contain –O–, –S– or a second N atom as hetero atom and may carry up to 4 substituents $R_1$ and $R_2$ in the nucleus; with the proviso that if A is morpholino or ethyl- or methyl-substituted morpholino, G may not likewise be morpholino or ethyl- or methyl-substituted morpholino; and
c) optionally further lubricants; said base oil (a) containing 0.001 to 5% by weight, based on the base oil, of a compound of the formula I.

2. A lubricant composition according to claim 1, wherein A and G in formula I are the same or different and represent

$$\begin{matrix}R^1\\R^2\end{matrix}{>}N-$$

wherein $R^1$ and $R^2$ are the same or different and are straight chain or branched $C_1$–$C_{24}$alkyl which is unsubstituted or substituted by –Cl, –$ZR^5$, –ZCN, –NCZ, –$CZZ^1R^5$, –$ZCZ^1R^5$, –$CZNR^7R^8$, –$NR^7R^8$ or –$CZZ^1E$, with the proviso that if only A has the meaning given above, G is a 5- to 12-membered heterocyclic radical as defined in claim 1.

3. A lubricant composition according to claim 1, wherein in formula I A represents morpholino and G represents

$$\begin{matrix}R^1\\R^2\end{matrix}{>}N-$$

wherein $R^1$ and $R^2$ are as defined in claim 1.

4. A lubricant composition according to claim 1, wherein A and G in formula I are the same or different and represent

$$\begin{matrix}R^1\\R^2\end{matrix}{>}N-$$

wherein $R^1$ and $R^2$ are the same or different and, in the same way as $R^5$, represent straight chain or branched $C_1$–$C_{18}$alkyl which is unsubstituted or substituted by –Cl, –$ZR^5$, ZCN, –$CZZ^1R^5$, $NR^7R^8$, –$ZCZ^1R^5$, –$CZNR^7R^8$ or –$CZZ^1E$, with the proviso that, if only A has the meaning given above, G is a 5- to 12-membered heterocyclic radical as defined in claim 1.

5. A lubricant composition according to claim 1, wherein A and G in formula I are the same or different and represent

$$\begin{matrix}R^1\\R^2\end{matrix}\!>\!N-$$

wherein $R^1$ and $R^2$ are the same or different and represent straight chain or branched, unsubstituted $C_1$–$C_{18}$alkyl.

6. A lubricant composition according to claim 5, wherein A and G in formula I represent

$$\begin{matrix}R^1\\R^2\end{matrix}\!>\!N-$$

wherein $R^1$ and $R^2$ are identical radicals and represent $C_5$–$C_{15}$alkyl.

7. A lubricant composition according to claim 1, wherein A and G in formula I are the same and represent an identical 5- to 12-membered heterocyclic radical as defined in claim 1.

8. A lubricant composition according to claim 1, wherein n in formula I is 2.

9. A lubricant composition according to claim 1, which contains as further lubricant additive (c) one selected from the group consisting of antioxidants, metal deactivators, rust inhibitors, dispersants, detergents, viscosity index improvers or pour-point depressors, or a mixture thereof.

10. Use of a compound of the formula I according to claim 1, as lubricant additive.

11. Use according to claim 10 of several compounds of the formula I as lubricant additives, which compounds are added to the base oil either singly or in admixture with one another.

**Revendications**

1. Compositions lubrifiantes qui contiennent:

a) une huile de base minérale et/ou synthétique,

b) un ou plusieurs composés répondant à la formule I:

$$A-(S)_n-G \qquad (I)$$

dans laquelle n désigne un nombre de 2 à 6, et A et G représentent chacun, indépendamment l'un de l'autre, un radical:

$$\begin{matrix}R^1\\R^2\end{matrix}\!>\!N-$$

dans lequel $R^1$ et $R^2$ représentent chacun, indépendamment l'un de l'autre, un alkyle en $C_1$–$C_{24}$ linéaire ou ramifié, un alcényle en $C_3$–$C_{18}$, un cycloalkyle en $C_3$–$C_{12}$, un aryle contenant de 6 à 10 atomes de carbone dans son noyau ou un aralkyle pouvant contenir jusqu'à 30 atomes de carbone (dans le dénombrement des atomes de carbone les substituants éventuels ne sont pas pris en compte), chacun de ces radicaux portant éventuellement un radical –Cl, $-ZR^5$, –ZCN, NCZ, $-CZZ^1R^5$, $-ZCZ^1R^5$, $-CZNR^7R^8$, $-NR^7R^8$ ou $-CZZ^1E$, Z et $Z^1$ représentant chacun, indépendamment l'un de l'autre, O, S, $S_2$, SO, $SO_2$ ou $SO_3$, $R^5$ représentant l'hydrogène ou un radical alkyle en $C_1$–$C_{18}$ ou alcényle en $C_3$–$C_{18}$ éventuellement porteur d'un radical –Cl, –ZCN, –NCZ ou –CN, et

E représentant Na, K ou un radical $-NR^7R^8R^9$ dans lequel $R^7$ à $R^9$ ont les significations qui ont été données ci-dessus pour $R^5$ et peuvent être identiques ou différents les und des autres,

ou dans lequel A et/ou G représente un radical hétérocyclique contenant de 5 à 12 maillons dans son cycle, cycle qui est saturé ou insaturé, qui contient un atome d'azote comme atome assurant la liaison avec le radical $-(S)_n-$ et qui peut en outre contenir O, S ou un second N comme hétéroatome et porter, comme substituants, jusqu'à 4 radicaux pris parmi ceux qui ont été définis pour $R^1$ et $R^2$, avec la condition que, lorsque A représente un radical morpholino ou un radical morpholino porteur d'un méthyle ou d'un éthyle, G ne peut pas être également un radical morpholino ou un radical morpholino porteur d'un méthyle ou d'un éthyle, et

c) éventuellement d'autres additifs pour lubrifiants, l'huile de base (a) contenant de 0.001 à 5% en poids, par rapport à elle-même, du composé de formule I.

2. Compositions lubrifiantes selon la revendication 1, caractérisées en ce que, dans la formule I, les symboles A et G représentent chacun, indépendamment l'un de l'autre, un radical de formule:

$$\begin{matrix}R^1\\R^2\end{matrix}\!>\!N-$$

dans lequel $R^1$ et $R^2$ représentent chacun, indépendamment l'un de l'autre, un radical alkyle en $C_1$–$C_{24}$, linéaire ou ramifié, éventuellement porteur d'un radical –Cl, $-ZR^5$, –ZCN, $-CZZ^1R^5$, $-ZCZ^1R^5$, $-CZNR^7R^8$, $-NR^7R^8$ ou $CZZ^1E$, ou A seul a la signification qui vient d'être donnée et G représente un radical hétérocyclique dont le noyau comporte de 5 à 12 maillons, tel que défini à la revendication 1.

3. Compositions lubrifiantes selon la revendication 1, caractérisées en ce que, dans la formule I, le symbole A représente un radical morpholino et le symbole G un radical:

$$\begin{matrix}R^1\\R^2\end{matrix}\!>\!N-$$

dans lequel $R^1$ et $R^2$ ont les significations qui leur ont été données à la revendication 1.

4. Compositions lubrifiantes selon la revendication 1, caractérisées en ce que, dans la formule I, les symboles A et G représentent chacun, indépendamment l'un de l'autre, un radical:

$$\begin{matrix}R^1\\R^2\end{matrix}\!>\!N-$$

dans lequel $R^1$ et $R^2$ sont identiques ou différents et représentent chacun, de même que $R^5$, un radical alkyle en $C_1$–$C_{18}$, linéaire ou ramifié, qui porte éventuellement un radical –Cl, $-ZR^5$, –ZCN, –NCZ, $-CZZ^1R^5$, $-NR^7R^8$, $-ZCZ^1R^5$, $-CZNR^7R^8$ ou $-CZZ^1E$, ou seulement A a la signification précédente et G

représente un radical hétérocyclique dont le noyau comporte de 5 à 12 maillons, tel que défini à la revendication 1.

5. Compositions lubrifiantes selon la revendication 1, caractérisées en ce que, dans la formule I, les symboles A et G représentent chacun, indépendamment l'un de l'autre, un radical:

$$\begin{matrix}R^1\\R^2\end{matrix}{>}N-$$

dans lequel $R^1$ et $R^2$ représentent chacun, indépendamment l'un de l'autre, un radical alkyle en $C_1$–$C_{18}$ linéaire ou ramifié qui ne porte pas de substituant.

6. Compositions lubrifiantes selon la revendication 5, caractérisées en ce que, dans la formule I, les symboles A et G représentent chacun un radical:

$$\begin{matrix}R^1\\R^2\end{matrix}{>}N-$$

dans lequel $R^1$ et $R^2$ représentent chacun le même radical alkyle contenant de 5 à 15 atomes de carbone.

7. Compositions lubrifiantes selon la revendication 1, caractérisée en ce que, dans la formule I, les symboles A et G représentent chacun le même radical pris parmi les radicaux cycliques dont le cycle comporte de 5 à 12 maillons, tel que défini à la revendication 1.

8. Compositions lubrifiantes selon la revendication 1, caractérisées en ce que, dans la formule I, l'indice n représente le nombre 2.

9. Compositions lubrifiantes selon la revendication 1, caractérisée en ce qu'elles contiennent, comme additifs supplémentaires (c), un additif pour lubrifiants qui appartient à l'ensemble constitué par les antioxydants, les désactivants de métaux, les inhibiteurs de corrosion, les dispersants, les détergents, les améliorateurs d'indice de viscosité et les abaisseurs de point de congélation, ou un mélange de plusieurs de ces substances.

10. Application de composés de formule I selon la revendication 1 comme additifs pour lubrifiants.

11. Application de plusieurs composés de formule I selon la revendication 10 comme additifs pour lubrifiants, les composés étant ajoutés à l'huile de base soit isolément soit en mélange.